(19)

Europäisches
Patentamt
European
Patent Office
Office européen
des brevets

(11) **EP 3 632 959 A1**

(12) **EUROPEAN PATENT APPLICATION**
published in accordance with Art. 153(4) EPC

(43) Date of publication:
**08.04.2020  Bulletin 2020/15**

(21) Application number: **18809275.3**

(22) Date of filing: **18.05.2018**

(51) Int Cl.:
*C08G 65/28* (2006.01)       *A61K 8/45* (2006.01)
*A61K 8/86* (2006.01)        *A61K 8/90* (2006.01)
*A61Q 5/00* (2006.01)        *A61Q 13/00* (2006.01)
*A61Q 15/00* (2006.01)       *A61Q 19/00* (2006.01)
*A61Q 19/10* (2006.01)       *B01F 1/00* (2006.01)
*B01F 17/42* (2006.01)       *C08K 5/01* (2006.01)
*C08K 5/10* (2006.01)        *C08L 71/02* (2006.01)
*C11B 9/00* (2006.01)

(86) International application number:
**PCT/JP2018/019389**

(87) International publication number:
**WO 2018/221278 (06.12.2018 Gazette 2018/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.05.2017   JP 2017105299
29.05.2017   JP 2017105301
18.10.2017   JP 2017202240
26.03.2018   JP 2018058818**

(71) Applicant: **NOF Corporation
Shibuya-ku
Tokyo 150-6019 (JP)**

(72) Inventors:
• **MURAI, Masaki**
  **Kawasaki-shi**
  **Kanagawa 210-0865 (JP)**
• **ICHIKAWA, Shoko**
  **Kawasaki-shi**
  **Kanagawa 210-0865 (JP)**
• **SEKIGUCHI, Koji**
  **Kawasaki-shi**
  **Kanagawa 210-0865 (JP)**
• **WAKITA, Kazuaki**
  **Kawasaki-shi**
  **Kanagawa 210-0865 (JP)**

(74) Representative: **Williams Powell
11 Staple Inn
London WC1V 7QH (GB)**

(54) **ALKYLENE OXIDE DERIVATIVES, WETTING AGENT, SOLUBILIZING AGENT, AND SOLUBILIZING COMPOSITION**

(57)    An alkylene oxide derivative represented by following formula (I):

$$R\text{-}O\text{-}(AO)_a\text{-}[(PO)_b/(EO)_c]\text{-}H \quad ... \quad (I)$$

(R represents a linear or branched alkyl group having from 4 to 36 carbons; AO is an oxyalkylene group having three or four carbons, PO is an oxypropylene group, and EO is an oxyethylene group; a, b, and c are the average addition molar numbers of the oxyalkylene group, the oxypropylene group, and the oxyethylene group per molecule, respectively, and $1 \leq a \leq 40$, $1 \leq b \leq 40$, $1 \leq c \leq 80$, and $(a + b + c) \geq 20$; $[(PO)_b/(EO)_c]$ represents a polyoxyalkylene group in which b moles of PO and c moles of EO are bonded randomly, and the random rate x is $0.1 \leq x < 1$).

EP 3 632 959 A1

**Description**

[Technical Field]

The present invention relates to a novel alkylene oxide derivative, a wetting agent, a solubilizing agent for solubilizing an oily component in water, and a solubilizing composition in which a certain saturated compound is solubilized. Specifically, the present invention relates to a novel alkylene oxide derivative capable of stably solubilizing an oily component for a long period of time in a liquid state at room temperature and having an excellent wetting effect; a solubilizing agent and a wetting agent comprising the alkylene oxide derivative; and a solubilizing composition in which a certain saturated compound is solubilized using the alkylene oxide derivative.

[Background Art]

There are various types of non-ionic surfactants, and an example of the non-ionic surfactants includes an alkylene oxide derivative. An alkylene oxide derivative is useful surfactants which allow for control of the balance between hydrophilicity and lipophilicity and the molecular mass by adjusting the addition molar numbers of oxyethylene groups, oxypropylene groups, and the like. Among these, polyoxyethylene alkyl ethers in which ethylene oxide is added to higher alcohols, have low critical micelle concentration and thus can be used in low concentration, and due to having low foamability, polyoxyethylene alkyl ethers are used in various industrial applications as detergents, emulsifying and solubilizing agents, wetting agents, penetrating agents, and lubricants.

In recent years, in the fields of pharmaceuticals, cosmetics, and toiletries, the following needs are increasing: a need to stably solubilize an oily component such as fragrances and oil-soluble active components at concentrations as high as possible in aqueous preparations; a need to stably solubilize, in aqueous preparations, an oily component that imparts lubricity in the field of lubricating oils; and a need to stably solubilize, in aqueous systems, oil-soluble colorants at high concentrations in the fields of ink and paint. In order to stably solubilize the oily component in water, it is necessary to increase the molecular mass of hydrophobic groups interacting with the oil.

However, the maximum molecular mass of general higher alcohols is about 500, and polyoxyethylene alkyl ethers having such hydrophobic groups have insufficient solubilizing power, and thus, when high amounts of polyoxyethylene alkyl ethers are blended to solubilize the oily component, the forming of foams may be a problem.

In view of these circumstances, PTL 1 and PTL 2, for example, describe non-ionic surfactants having excellent surface active property such as solubilization and emulsification, in which the non-ionic surfactants are obtained by further adding ethylene oxide, after increasing the molecular mass of the hydrophobic group by adding butylene oxide to an alcohol having large molecular mass such as 2-decyltetradecanol or dimer diol.

However, these non-ionic surfactants have a large molecular mass of hydrophobic groups, and thus, to impart water solubility, it is also necessary to increase the molecular mass of polyethylene glycol chains (PEG chains) that are hydrophilic groups. Therefore, the crystallinity of the PEG chains increases and makes the non-ionic surfactants solid at room temperature, and thus, there are problems in that the handleability is poor, and that wettability, which is an essential function of a surfactant, decreases.

Further, PTL 3, for example, describes an alkylene oxide adduct, into which a random copolymerization of ethylene oxide and propylene oxide is introduced to reduce crystallinity, having excellent low foamability and antifoaming properties.

However, this surfactant was not satisfactory in terms of solubilizing power to an oily component.

Thus, there is a desire for an alkylene oxide derivative which is liquid at room temperature, can stably solubilize an oily component for a long period of time, and has an excellent wetting effect.

On the other hand, when an oily component is solubilized, problems resulting from the characteristics of the oily component may arise. Specifically, when the oily component is solubilized, since many of the commonly used solubilizing agents are solid at room temperature, the number of steps for dissolving the solubilizing agents may increase. Further, since the oily component may be solubilized while being heated, depending on the type of the oily component, blending may be difficult. For example, when volatile fragrances are solubilized, the fragrance may volatilize, heat-sensitive unsaturated compounds may be decomposed or oxidized by heating, and thus, long-term stability may decrease. Further, saturated compounds having large partition coefficients are difficult to be solubilized, and it is difficult to obtain a stable and highly transparent liquid.

Therefore, it is usually necessary to use a large amount of the solubilizing agent with respect to an oily component to obtain a highly transparent solution. As a result, after solubilization, the amount of the solubilizing agent around the oily component increases, which may lead to the suppression of fragrance development of the fragrance and stickiness during application due to the solubilizing agent, and further, the concentration of the solubilizing agent in the preparation increases due to volatilization of water, which may lead to problems such as deterioration of long-term stability.

In response to such problems, PTL 4, for example, discloses, as a solubilization method offering excellent stability of oily components, fragrance retention, and feeling in use, the combined use of polyoxyethylene (5-10) coconut oil fatty acid glyceryl and/or polyoxyethylene (5-10) coconut (caprylic acid/capric acid) glyceryl with polyoxyethylene (30-80).

However, it is necessary to impart heat when the oily components are solubilized with this combination, and thus, fragrance components may volatilize during preparation. Further, heat-sensitive unsaturated compounds such as vitamin A may be decomposed or oxidized during preparation, further leading to a decrease in long-term stability. In addition, there is also a problem in that it is difficult to deal with a low-energy cold process requiring no heating.

Further, PTL 5, for example, describes external preparations such as cosmetics containing an alkylene oxide derivative, water, and an oily component. PTL 5 states that the temporal stability of the external preparations is improved, and in particular, the external preparations are stable against temperature change over time.

However, the blending amount of the alkylene oxide derivative with respect to the oily component is too large, and stickiness may occur during application on the skin. Further, there is an increase in prescriptions of preparations using a large amount of an oily component such as fragrances, saturated compounds that have large partition coefficients and are difficult to be solubilized, vitamin E and its derivatives, however, the technique in PTL 5 may not satisfy the requirements for solubilization.

[Citation List]

[Patent Literature]

[PTL 1] Japanese Unexamined Patent Application Publication No. 2000-325771 A
[PTL 2] Japanese Unexamined Patent Application Publication No. 2008-239496 A
[PTL 3] Japanese Unexamined Patent Application Publication No. 2014-240378 A
[PTL 4] Japanese Unexamined Patent Application Publication No. 2001-213718 A
[PTL 5] Japanese Unexamined Patent Application Publication No. 2008-266217 A

[Summary of Invention]

[Technical Problem]

As described above, an object of the present invention is to provide an alkylene oxide derivative capable of stably solubilizing an oily component for a long period of time and having an excellent wetting effect and handleability in a liquid state at room temperature.

Further, another object of the present invention is to provide a wetting agent having an effect of changing the surface tension between a solid and a liquid to make the surface of the solid easy to wet.

Further, another object of the present invention is to provide a solubilizing agent that is liquid at room temperature, is capable of solubilizing, in a cold process and with the use of a small amount, an oily component such as a fragrance, an unsaturated compound, and a certain saturated compound in water, provides low stickiness during application, offers excellent long-term stability of solubilized solutions of an oily component, and in particular, provides excellent fragrance development of the solution of the fragrance if the oily component is a fragrance.

Moreover, another object of the present invention is to provide a solubilizing composition which is capable of solubilizing, in a cold process and with the use of a small amount, a certain saturated compound in water, provides low stickiness during application, and offers excellent long-term stability of the solubilized solution.

[Solution to Problem]

As a result of diligent studies to achieve the objects described above, the present inventors have found that the objects described above can be achieved by using a novel alkylene oxide derivative and by using the novel alkylene oxide derivative, a certain saturated compound, a certain alkylene oxide derivative, and a certain glycerol monoalkyl ether in a predetermined blending ratio. The present invention based on these finding is as follows.

That is, the present invention provides an alkylene oxide derivative represented by the following formula (I):

$$R\text{-}O\text{-}(AO)_a\text{-}[(PO)_b/(EO)_c]\text{-}H \dots \qquad (I)$$

(R represents a linear or branched alkyl group having from 4 to 36 carbons; AO is an oxyalkylene group having three or four carbons, PO is an oxypropylene group, and EO is an oxyethylene group; a, b, and c are the average addition molar numbers of the oxyalkylene group, the oxypropylene group, and the oxyethylene group per molecule, respectively, and $1 \leq a \leq 40$, $1 \leq b \leq 40$, $1 \leq c \leq 80$, and $(a + b + c) \geq 20$; $[(PO)_b/(EO)_c]$ represents a polyoxyalkylene group in which b moles of PO and c moles of EO are bonded randomly, and the random rate x is $0.1 \leq x < 1$).

Further, the present invention provides a wetting agent comprising the alkylene oxide derivative according to the present invention.

Further, the present invention provides a solubilizing agent for solubilizing an oily component in water and the solubilizing agent comprises the alkylene oxide derivative according to the present invention.

The oily component is preferably a fragrance, an unsaturated compound, or a saturated compound having an octanol/water partition coefficient of 7 to 20 and a molar mass of 250 g/mol or more.

Moreover, the present invention provides a solubilizing composition containing 0.01 to 5 mass% of component (A), 0.1 to 10 mass% of component (B), 0.1 to 20 mass% of component (C), and 0.01 to 5 mass% of component (D), with the ratio of the mass of component (B) to the mass of component (A) (the mass of component (B)/the mass of component (A)) being 0.5 to 10 wherein

the component (A) is a saturated compound having an octanol/water partition coefficient of 7 to 20 and a molar mass of 250 g/mol or more,

the component (B) is the alkylene oxide derivative according to the present invention,

the component (C) is an alkylene oxide derivative represented by the following formula (II) where a 5 mass% aqueous solution of the component (C) has a cloud point of 15°C or higher

$$Z\text{-}\{O\text{-}[(PO)_p/(EO)_q]\text{-}(BO)_r\text{-}H\}_s \ldots \qquad \text{(II)}$$

(wherein Z is a residue obtained by removing all hydroxyl groups from a polyhydric alcohol having from two to seven carbons and containing s hydroxyl groups, and $2 \leq s \leq 4$; PO is an oxypropylene group, EO is an oxyethylene group, BO is an oxyalkylene group having four carbons, p, q, and r are the average addition molar numbers of the oxypropylene group, the oxyethylene group, and the oxyalkylene group per molecule, respectively, $1 \leq p \leq 30$, $1 \leq q \leq 30$, $1 \leq r \leq 2$, and PO and EO are bonded randomly), and

the component (D) is an alkyl glyceryl ether comprising glycerol and a monohydric alcohol including a branched alkyl having from seven to nine carbons.

[Advantageous Effects of Invention]

The alkylene oxide derivative according to the present invention can stably solubilize an oily component for a long period of time, and has an excellent wetting and penetrating effect and excellent handleability in a liquid state at room temperature.

With the wetting agent comprising the alkylene oxide derivative of the present invention, the surface wettability can be improved in various solids such as glass, metal, and resin.

The solubilizing agent comprising the alkylene oxide derivative of the present invention is liquid at room temperature, can solubilize, in a cold process and with the use of a small amount, an oily component in water, provides low stickiness during application, and can provide a solution offering excellent long-term stability. Further, if the oily component is a fragrance, an effect is obtained in which an excellent fragrance development of the solubilized solution of the fragrance is achieved.

According to a solubilizing composition of the present invention, it is possible to obtain a solubilizing composition which is capable of solubilizing, in a cold process and with the use of a small amount, a certain saturated compound in water, provides low stickiness during application, and offers excellent long-term stability of the solubilized solution.

[Description of Embodiments]

An alkylene oxide derivative according to the present invention, a solubilizing agent comprising the alkylene oxide derivative according to the present invention, and a solubilizing composition containing the alkylene oxide derivative according to the present invention will be described successively below.

Note that, in the present invention, "solubilization" refers to a technique allowing for preparation of a highly stable and transparent solution from an oily component in the presence of an aqueous component and a solubilizing agent. The "aqueous component" refers to water; lower alcohols such as ethyl alcohol and propyl alcohol; mixed liquids of water and lower alcohols; and the like.

Further, the "oily component" refers to a component that does not dissolve or is difficult to dissolve in an aqueous component, and includes, for example, at least a fragrance, an unsaturated compound, and a certain saturated compound described later.

Further, in the present invention, "liquid at room temperature" means being in a liquid state at 25°C.

Alkylene Oxide Derivative

An alkylene oxide derivative according to the present invention is represented by the following formula (I):

$$R\text{-}O\text{-}(AO)_a\text{-}[(PO)_b/(EO)_c]\text{-}H \dots \qquad (I).$$

R represents a linear or branched alkyl group having from 4 to 36 carbons. The lower limit of the number of the carbons is preferably 12, particularly preferably 18. The upper limit of the number of the carbons is preferably 32, particularly preferably 26, and more preferably 24. Preferable combinations of the upper limit and the lower limit of the number of the carbons include, for example, 4 to 24, 12 to 24, 12 to 32, and 18 to 26.

If R has less than four carbons, the solubilizing power decreases. If R has too many carbons, the crystallinity increases and the solution may become solid at room temperature or the wetting effect may decrease. Further, if R has too many carbons, stickiness may occur during application of the composition in which the oily component is solubilized.

R is derived from alcohol used in production of the alkylene oxide derivative of formula (I). The alcohol is a linear or branched alcohol having from 4 to 36 carbons.

Examples of the alcohol include linear alcohols such as butanol, pentanol, hexanol, heptanol, octanol, nonanol, decanol, undecanol, dodecanol, tridecanol, tetradecanol, pentadecanol, hexadecanol, heptadecanol, octadecanol, nonadecanol, eicosanol, docosanol, tetracosanol, hexacosanol, octacosanol, triacosanol, and hexatriacosanol; branched alcohols such as 2-ethylhexanol, 2-hexyloctanol, 1-methylheptadecanol, isodecanol, 2-octyldecanol, 2-decyltetradecanol, 2-tetradecyloctadecanol, and 2-hexadecyleicosanol; linear alkenols such as hexadecenol, octadecenol, eicosenol, and docosenol; and branched alkenols such as 1-methylheptadecenol and isotridecenol. One or more types of these alcohols may be used.

AO is an oxyalkylene group having three or four carbons, and examples thereof include an oxypropylene group, an oxyisobutylene group, an oxy-1-ethylethylene group, an oxy-2-butylene group, and an oxytetramethylene group.

If the average addition molar number a of AO is two or more, the two or more AOs may be from the same group or from different groups. When the AOs are from two or more different groups, either random addition or block addition may be used.

PO is an oxypropylene group and EO is an oxyethylene group. In formula (I), a, b, and c are the average addition molar numbers of AO, PO, and EO per molecule, respectively.

a is a number of 1 to 40. The upper limit of a is preferably 30, particularly preferably 20, and more preferably 13. The lower limit of a is preferably 2, particularly preferably 3, and more preferably 4. Preferable combinations of the upper limit and the lower limit of a include, for example, 3 to 30, 4 to 20, 1 to 30, 2 to 20, and 3 to 13.

If a is too small, the solubilizing power decreases, and if a is too large, the wetting effect may decrease, and further, stickiness may occur during application of the composition in which the oily component is solubilized.

b is a number of 1 to 40. The upper limit of b is preferably 30, particularly preferably 20, and more preferably 10. The lower limit of b is preferably 2, particularly preferably 3, and more preferably 4. Preferable combinations of the upper limit and the lower limit of b include, for example, 3 to 30, 4 to 20, 1 to 30, 2 to 20, and 3 to 10.

If b is too small, the alkylene oxide derivative becomes solid at room temperature and the handleability may decrease, and further, the solubilizing power may decrease. If b is too large, the solubilizing power may decrease, and further, stickiness may occur during application of the composition in which the oily component is solubilized.

c is a number of 1 to 80. The upper limit of c is preferably 60, particularly preferably 40, and more preferably 30. The lower limit of c is preferably 5, and particularly preferably 10. Preferable combinations of the upper limit and the lower limit of c include, for example, 5 to 60, 10 to 40, 1 to 60, 5 to 40, and 10 to 30.

If c is too small, the solubilizing power may decrease. If c is too large, the crystallinity increases and the alkylene oxide derivative becomes solid at room temperature, solubilization may not be possible in a cold process without heating, and further, the wetting effect may decrease.

(a + b + c) is 20 or more, preferably 30 or more, and particularly preferably 35 or more. If (a + b + c) is less than 20, the solubilizing power may decrease. Further, from the viewpoint of ease of handling, (a + b + c) is preferably 100 or less, particularly preferably 80 or less, more preferably 50 or less, and even more preferably 40 or less.

The bonding moiety of EO and PO in formula (I) is described as $[(PO)_b/(EO)_c]$, and this description indicates that, in the present invention, b moles of PO and c moles of EO are bonded randomly, and not in blocks. If EO and PO are bonded in blocks, the alkylene oxide derivative becomes solid at room temperature, the wetting effect decreases, and the solubilizing power may also decrease.

Further, if R has n carbons, a y value represented by $y = (2n + a)$ is preferably $30 \le y \le 100$, particularly preferably $35 \le y \le 80$, and more preferably $40 \le y \le 60$.

On the other hand, from the viewpoint of wettability, the y value represented by $y = (2n + a)$ is preferably $20 \le y \le 100$, particularly preferably $25 \le y \le 75$.

The alkylene oxide derivative represented by formula (I) according to the present invention can be produced by a known method. For example, after addition polymerization of an alkylene oxide to a linear or branched alkyl alcohol having from 4 to 36 carbons in the presence of an alkali catalyst at 50 to 160°C and at 0.5 MPa (gauge pressure) or less, an alkylene oxide derivative can be obtained by additionally polymerizing a mixture of ethylene oxide and propylene oxide, neutralizing the resultant with an acid such as hydrochloric acid, phosphoric acid, or acetic acid, and removing the water content and the salt from the neutralization.

If the random rate of the alkylene oxide derivative represented by formula (I) according to the present invention is x, x is $0.1 \leq x < 1$, preferably $0.3 \leq x \leq 0.97$, particularly preferably $0.5 \leq x \leq 0.9$, more preferably $0.6 \leq x \leq 0.85$, and even more preferably $0.7 \leq x \leq 0.8$.

If the random rate x is less than 0.1, the alkylene oxide derivative may have solid properties at room temperature, and the solubilizing ability and the wetting effect may decrease.

The random rate in the alkylene oxide derivative represented by formula (I) can be determined by the following formula (III) from each of the average addition molar numbers a, b, and c of AO and EO per molecule in formula (I).

$$x = (b + c)/(a + b + c) \quad (III)$$

Wetting Agent

The wetting agent according to the present invention comprises the alkylene oxide derivative represented by formula (I) of the present invention.

The wetting agent according to the present invention can be used as a preparation in which the alkylene oxide derivative represented by formula (I) is dissolved in an aqueous component. Water is preferred as the aqueous component.

The content of the alkylene oxide derivative in the wetting agent according to the present invention is preferably 0.1 to 10 mass%, particularly preferably 0.5 to 8 mass%, and more preferably 1 to 5 mass%. If the content of the alkylene oxide derivative is too small, the wetting effect may be insufficient. Conversely, if the content is too high, foaming may occur.

The wetting agent according to the present invention may contain, as other optional components, components generally used in cosmetics.

Solubilizing Agent

The solubilizing agent according to the present invention is a solubilizing agent for solubilizing an oily component in water and comprises the alkylene oxide derivative represented by formula (I) of the present invention.

The oily component solubilized by the solubilizing agent according to the present invention is not particularly limited as long as the oily component does not dissolve or is difficult to dissolve in the aqueous component, and, from the viewpoint of the effect, a fragrance, an unsaturated compound, and a certain saturated compound are preferred. Below, the fragrance, unsaturated compound, and certain saturated compound are described.

Fragrance

Examples of the fragrance include natural fragrances obtained from animals or plants, synthetic fragrances produced by a chemical compatibility means, and blended fragrances that are mixtures thereof, however, the fragrance is not particularly limited to these fragrances. In the present invention, any one or more selected from these fragrances can be used in accordance with the intended product.

Examples of the fragrance to be used include hydrocarbons such as limonene, alcohols such as 1-nonanol, phenols, aldehydes such as p-anisaldehyde, fatty acids such as caprylic acid, ketones, acetals, ethers such as anethole, esters such as ethyl hexanoate, carbonates, lactones, oximes, nitriles, Schiff bases, amides, nitrogen-containing compounds, sulfur-containing compounds, natural essential oils, and natural extracts.

Unsaturated Compound

Examples of the unsaturated compound include oil-soluble vitamins, oil-soluble derivatives of water-soluble vitamins, and unsaturated fatty acids.

Examples of the oil-soluble vitamins and the oil-soluble derivatives of water-soluble vitamins include vitamin A and its derivatives, vitamin B2 derivatives, vitamin B6 derivatives, vitamin D and its derivatives, vitamin E and its derivatives, essential fatty acids, ubiquinones and their derivatives, vitamin Ks, resorcinol derivatives, glycyrrhetinic acid and its derivatives, and oil-soluble vitamin C derivatives, however, the oil-soluble vitamins and the oil-soluble derivatives of water-soluble vitamins are not particularly limited to these examples. In the present invention, any one or more selected from these vitamins and their derivatives can be used in accordance with the intended product.

Examples of the unsaturated fatty acid include undecylenic acid, myristoleic acid, palmitoleic acid, oleic acid, elaidic acid, vaccenic acid, linoleic acid, linolenic acid, pinolenic acid, stearidonic acid, eleostearic acid, bosseopentaenoic acid, docosahexaenoic acid, eicosapentaenoic acid, and arachidonic acid, however, the unsaturated fatty acid is not particularly limited to these examples. In the present invention, any one or more of these unsaturated fatty acids can be used in accordance with the intended product.

Saturated Compound

The saturated compound in the present invention has an octanol/water partition coefficient of 7 to 20. In the present invention, any one or more types of the saturated compound can be used in accordance with the intended product.

The octanol/water partition coefficient is one of the numerical values expressing the properties of chemical substances, and is a constant value not depending on the added amount. In a mixed liquid in equilibrium at the interface between a water phase and an n-octanol phase, the octanol/water partition coefficient is the ratio between the concentration of the target substance dissolved in the water phase and the concentration of the target substance dissolved in the n-octanol phase, represented by a common logarithm that is generally expressed by $Log P_{ow}$. If the partition coefficient increases, the hydrophobicity tends to increase proportionally, and if the partition coefficient decreases, the hydrophilicity tends to increase proportionally.

Note that the octanol/water partition coefficient ($Log P_{ow}$) in the present invention is calculated by a calculation method known in the art. Further, the partition coefficient ($Log P_{ow}$) may also be a value determined by a calculation employing commercially available software and the like using a quantitative structure-activity correlation algorithm. An example of commercially available softwares capable of calculating the partition coefficient ($Log P_{ow}$) includes Chemdraw Pro 12.0, and further, http://www.vcclab.org/lab/alogps/start.html may be utilized to obtain values for the partition coefficient.

The octanol/water partition coefficient is preferably from 10 to 20, particularly preferably from 10 to 15.

Further, the molar mass of the saturated compound is 250 g/mol or more, preferably 300 to 600 g/mol, and particularly preferably 400 to 500 g/mol.

Note that the molar mass of the saturated compound in the present invention is determined from the molecular mass of the saturated compound. The molecular mass of the saturated compound can be determined by common analytical methods such as gel permeation chromatography (GPC) with polystyrene as standard and tetrahydrofuran (THF) as developing solvent, or mass spectrometry.

As the saturated compound in the present invention, a compound satisfying the above-described characteristics can be used, however, from the viewpoint of obtaining a prominent effect in the present invention, hydrocarbon compounds and ester compounds are preferred, and hydrocarbon compounds are particularly preferred.

Specific examples of these hydrocarbon compounds include isododecane, isohexadecane, hydrogenated polyisobutene, light liquid isoparaffin, liquid paraffin, squalane, and petroleum jelly, and liquid paraffin and squalane are preferred, and squalane is particularly preferred.

Further, specific examples of the ester compounds include lauryl laurate, palmityl palmitate, stearyl stearate, myristyl myristate, cetyl 2-ethylhexanoate, and octyldodecyl oleate.

The used amount of the solubilizing agent according to the present invention with respect to the oily component is, in terms of the ratio of the mass of the solubilizing agent to the mass of the oily component, preferably in the range of 1 to 7, particularly preferably in the range of 2 to 5, and more preferably in the range of 3 to 5. If this mass ratio is too small, the fragrance development of the fragrance may be weakened and the long-term stability of the unsaturated compound may decrease. Conversely, if this mass ratio is too large, stickiness may occur during application.

The content of the solubilizing agent in the preparation containing the solubilizing agent of the present invention and the oily component is preferably 0.1 to 10 mass%, particularly preferably 0.5 to 8 mass%, and more preferably 1 to 5 mass%. If the content of the solubilizing agent is too small, solubilization of the oily component is difficult, the fragrance development of the fragrance may be weakened and the long-term stability may decrease. Conversely, if the content of the solubilizing agent is too high, stickiness may occur during application.

Other Optional Components

The solubilizing agent according to the present invention may contain, as other optional components, components generally used in cosmetics.

Solubilizing Composition

The solubilizing composition according to the present invention contains component (A), component (B), component (C), component (D), water, and other optional components.

Since component (A) is the oily component and component (B) is the solubilizing agent according to the present invention, description of these components will be omitted, whereas the other components will be described below.

Component (C): Alkylene Oxide Derivative

In the present invention, an alkylene oxide derivative represented by the following formula (II) is used as component (C). Only one type of component (C) may be used or two or more types of component (C) may be used together. By combining component (B), component (C), and component (D), component (A) can be solubilized by component (B) in a low concentration. As a result, a solubilizing composition with little stickiness and excellent long-term stability can be obtained.

$$Z\text{-}\{O\text{-}[(PO)_p/(EO)_q]\text{-}(BO)_r\text{-}H\}_s \ldots \qquad (II)$$

Z in formula (II) is a residue obtained by removing all hydroxyl groups from a polyhydric alcohol having two to seven carbons and containing s hydroxyl groups, in which s is a number of 2 to 4. The polyhydric alcohol having from two to seven carbons and containing two to four hydroxyl groups is preferably a hydrocarbon containing hydroxyl groups, particularly preferably a saturated hydrocarbon containing hydroxyl groups.

Further, alkylglucosides having from one to two carbons are particularly preferable as the polyhydric alcohol having from two to seven carbons and containing two to four hydroxyl groups. Note that, "alkylglucosides having from one to two carbons" means ethers in which a hydrogen atom of one hydroxyl group among the hydroxyl groups of glucose is substituted with an alkyl group having from one to two carbons. Methylglucoside is particularly preferable as the alkylglucoside having from one to two carbons.

Z in formula (II) is preferably a residue obtained by removing a hydroxyl group from ethylene glycol, propylene glycol, glycerol, trimethylolpropane, pentaerythritol, or methylglucoside, particularly preferably, a residue obtained by removing a hydroxyl group from ethylene glycol, propylene glycol, glycerol, or trimethylolpropane, and more preferably, a residue obtained by removing a hydroxyl group from glycerol.

s in formula (II) is preferably a number of 2 to 3. If s is a number of 2 to 3, the long-term stability of the solubilizing composition is further improved.

PO in formula (II) is an oxypropylene group, EO is an oxyethylene group, and BO is an oxyalkylene group having four carbons. Examples of the oxyalkylene group BO having four carbons include, additionally to an oxy-(1-ethyl)ethylene group derived from 1,2-butylene oxide, an oxy-(1,1-dimethyl)ethylene group and a tetramethylene group, however, the oxy-(1-ethyl)ethylene group is preferred.

p, q, and r in formula (II) are the average addition molar numbers of the oxypropylene group, the oxyethylene group, and the oxyalkylene group per molecule, respectively.

p is a number of 1 to 30, preferably a number of 1 to 20, and particularly preferably a number of 1 to 10. If p is less than 1, the long-term stability of the solubilizing composition may decrease, and if p is too large, stickiness may occur during application of the solubilizing composition.

q is a number of 1 to 30, preferably a number of 1 to 20, and particularly preferably a number of 1 to 10. If q is less than 1, the long-term stability of the solubilizing composition may decrease, and if q is too large, stickiness may occur during application of the solubilizing composition.

r is a number of 1 to 2, and preferably 1. If r exceeds 2, the long-term stability of the solubilizing composition may decrease.

When the sum of the content of PO and the content of EO is considered as 100 parts by mass, the amount of PO in formula (II) is preferably from 30 to 90 parts by mass, particularly preferably from 30 to 60 parts by mass, and more preferably from 40 to 50 parts by mass. If the amount of PO is 30 parts by mass or more, the long-term stability of the solubilizing composition improves. If the amount of PO is 90 parts by mass or less, stickiness can be easily suppressed during application of the solubilizing composition. If the amount of PO is within the preferred range mentioned above, a solubilizing composition having a particularly good balance of long-term stability and stickiness during application can be obtained.

The bonding moiety of PO and EO in formula (II) is described as $[(PO)_p/(EO)_q]$, and this description indicates that PO and EO are bonded randomly, and not in blocks. Further, since this description indicates that PO and EO are bonded randomly, $Z\text{-}\{O\text{-}[(PO)_p/(EO)_q]$ in formula (II) not only expresses an alkylene oxide derivative in which PO is bonded to the "Z- {O-" moiety, but also inclusively expresses an alkylene oxide derivative in which EO is bonded to the "Z- {O-" moiety.

Unlike component (C) in the present invention, if an alkylene oxide derivative in which PO and EO are bonded in blocks is used, the long-term stability of the solubilizing composition may decrease and further, stickiness may occur during application of the solubilizing composition.

The alkylene oxide derivative represented by formula (II) according to the present invention can be produced by a known method. For example, the alkylene oxide derivative can be obtained by addition polymerization of ethylene oxide, propylene oxide, and alkylene oxides to a polyhydric alcohol having from two to seven carbons and containing two to four hydroxyl groups.

The cloud point of a 5 mass% aqueous solution of component (C) in the present invention is 15°C or higher, preferably 20°C or higher, particularly preferably 25°C or higher, and more preferably 35°C or higher. If the cloud point of the 5 mass% aqueous solution of component (C) is too low, the long-term stability of the solubilizing composition may decrease. Note that the upper limit of the cloud point of the 5 mass% aqueous solution of component (C) is usually 80°C.

The cloud point is defined in JIS K 3211 "Technical terms for surface active agents" as a "temperature at which cloudiness starts to occur when the temperature of an aqueous surfactant solution is increased" and is measured by the following method. After a 5 mass% aqueous solution of component (C) is poured in a test tube to a height of about 40 mm, a thermometer is put into the test tube, and while being stirred well with the thermometer, the solution is heated to about 2-3°C higher than the temperature at which cloudiness occurs. Then, the solution is cooled by air-cooling while being stirred well again, and the temperature at which the solution becomes transparent is measured as the cloud point. Further, if the aqueous solution is cloudy at room temperature, the aqueous solution is stirred well to be cooled until the aqueous solution becomes transparent, the aqueous solution is heated gradually, while being stirred well again, to the temperature at which turbidity occurs, and then the aqueous solution is cooled gradually while being stirred. The temperature at which the solution becomes transparent is measured as the cloud point

Component (D): Glyceryl Mono (C7-9 Branched Alkyl) Ether

In the present invention, a glyceryl mono(C7-9 branched alkyl) ether is used as component (D). Here, "glyceryl mono(C7-9 branched alkyl) ether" signifies an ether in which a hydrogen atom of one hydroxyl group of glycerol is replaced with a C7-9 branched alkyl group. Further, in the present description, "Cd-e" and "Cf" (d, e, and f indicate numbers) signify "having from d to e carbons" and "having f carbons", respectively. Only one type of component (D) may be used or two or more types of component (D) may be used together.

The glyceryl mono (C7-9 branched alkyl) ether can be produced, for example, from glycerol and a monohydric alcohol including a branched alkyl having from seven to nine carbons. Further, a commercially available glyceryl mono(C7-9 branched alkyl) ether can be used. Examples of the glyceryl mono(C7-9 branched alkyl) ether include ethylpentylglycerol, ethylhexylglycerol, and ethylheptylglycerol. Among these, ethylhexylglycerol is preferable from the viewpoint of the solubilized state and the long-term stability of the solubilizing composition. Note that an example of commercially available ethylhexylglycerol includes 3-(2-ethylhexyloxy)-1,2-propanediol manufactured by SACHEM, Inc.

Water

In the solubilizing composition according to the present invention, the saturated compound of component (A) is solubilized in water, and thus, the solubilizing composition contains water as an essential component. Examples of the water include ion-exchanged water, distilled water, RO water, tap water, and industrial water.

Content of Each Component

The content of component (A) in the solubilizing composition according to the present invention is 0.01 to 5 mass%, preferably 0.05 to 4 mass%, and particularly preferably 0.1 to 3 mass%. If the content of component (A) is too large, the long-term stability of the solubilizing composition may decrease.

The content of component (B) in the solubilizing composition according to the present invention is 0.1 to 10 mass%, preferably 0.5 to 8 mass%, and particularly preferably 1 to 5 mass%. If the content of component (B) is too large, stickiness may occur during application of the solubilizing composition, and if the content of component (B) is too low, solubilization of component (A) is difficult and the long-term stability of the solubilizing composition may decrease.

The ratio of the mass of component (B) to the mass of component (A) (the mass of component (B)/the mass of component (A)) is 0.5 to 10, preferably 1 to 8. If this mass ratio is too low, the long-term stability of the solubilizing composition may decrease, and if this mass ratio is too large, stickiness may occur during application of the solubilizing composition.

The content of component (C) in the solubilizing composition according to the present invention is 0.1 to 20 mass%, preferably 0.5 to 10 mass%, particularly preferably 1 to 8 mass%, and more preferably 2 to 6 mass%. If the content of component (C) is too large, stickiness may occur during application of the solubilizing composition.

The content of component (D) in the solubilizing composition according to the present invention is 0.01 to 5 mass%, preferably 0.1 to 3 mass%, and particularly preferably 0.3 to 1 mass%. If the content of component (D) is too low, the stability of the solubilizing composition may decrease, and conversely, if the content of component (D) is too large, stickiness may occur during application of the solubilizing composition.

Water is used in an amount to adjust the components (A) to (D) and optionally contained components to a predetermined amount. Specifically, the content of water in the solubilizing composition according to the present invention is 60 to 99.74 mass%, preferably 70 to 95 mass%, and particularly preferably 75 to 92 mass%.

Other Suitable Components

An even more excellent effect can be obtained when the solubilizing composition according to the present invention contains polyhydric alcohols or higher alcohols. Only one among a polyhydric alcohol and a higher alcohol may be used, however, if both a polyhydric alcohol and a higher alcohol are combined, an even more excellent effect can be obtained.

The polyhydric alcohol is a dihydric or higher alcohol, preferably a dihydric or trihydric alcohol. The polyhydric alcohol has, for example, from two to ten carbons, and preferably two to six carbons. Examples of the polyhydric alcohol include ethylene glycol, propylene glycol, dipropylene glycol, 1,3-butylene glycol, and glycerol, and 1,3-butylene glycol and glycerol are preferred.

The content of the polyhydric alcohol in the solubilizing composition according to the present invention is preferably 1 to 40 mass%, particularly preferably 10 to 30 mass%, and more preferably 15 to 25 mass%.

Examples of the higher alcohol include alcohols having from 12 to 22 carbons such as linear saturated alcohols such as lauryl alcohol, myristyl alcohol, cetanol, stearyl alcohol, and behenyl alcohol; branched saturated alcohols such as isostearyl alcohol and octyldodecanol; and unsaturated alcohols such as oleyl alcohol, and among these, branched saturated alcohols such as isostearyl alcohol and octyldodecanol are preferred.

The content of the higher alcohol in the solubilizing composition according to the present invention is preferably 0.01 to 10 mass%, particularly preferably 0.1 to 5 mass%, and more preferably 0.5 to 2 mass%.

Other Optional Components

The solubilizing composition according to the present invention may contain, as other optional components, components generally used in cosmetics and the like.

[Examples]

Below, the present invention will be described in more detail with reference to examples and comparative examples. Note that, in the following, "%" means "mass%" unless otherwise specified.

Synthesis Example 1: Synthesis of Compound 1

50 g of butanol and 2.5 g of potassium hydroxide as a catalyst were put into an autoclave, and after the air in the autoclave was replaced with dry nitrogen, the catalyst was dissolved at 140°C while being stirred. Subsequently, 1317 g of propylene oxide was added dropwise by a droplet dispensing device at 120°C and at 0.2 to 0.5 MPa (gauge pressure), and was stirred for three hours. Subsequently, a mixture of 1155 g of ethylene oxide and 247 g of propylene oxide was added dropwise by the droplet dispensing device at 120°C and at 0.2 to 0.5 MPa (gauge pressure), and was stirred for two hours. Afterwards, the reaction composition was taken out of the autoclave, neutralized with hydrochloric acid to pH 6 to 7, and treated at -0.095 MPa of reduced pressure (gauge pressure) and at 100°C for one hour to remove the contained water content. Further, filtration was performed to remove the salt produced after the treatment, and thus, compound 1 was obtained.

Further, the number average molecular mass of ethylene oxide and propylene oxide adducts was determined from the hydroxyl number obtained by "Determination of hydroxyl number" according to JIS K1557-1, and from the number average molecular mass, the values of a, b, and c in formula (I) were obtained.

Synthesis Example 2: Synthesis of Compounds 2 to 16

An alkylene oxide derivative was obtained as compound 2 by a method similar to Synthesis Example 1, except that the starting material and the addition amounts of ethylene oxide and propylene oxide were changed and 1,2-butylene oxide was used as the alkylene oxide. Compounds 3 to 16 were synthesized by similar synthesis methods. Table 1 shows the type of starting material and alkylene oxide, the carbon number n of the starting material, the representative average addition molar numbers (a, b, and c) of the alkylene oxide, ethylene oxide, and propylene oxide, the sum of the average addition molar numbers (a + b + c), the y value determined from (2n + a), and the random rate x. Further, Table 1 also shows compounds 17 to 19 used in the following comparative examples.

Compound 18 and Compound 19

Compound 18: Polyoxyethylene (60) hydrogenated castor oil ("Uniox HC-60" manufactured by NOF Corporation, label name: PEG-60 hydrogenated castor oil, freezing point: 32°C)
Compound 19: Polyoxyethylene sorbitan monooleate (20 E.O.) ("Nonion OT-221" manufactured by NOF Corporation, label name: polysorbate 80, freezing point: -20°C or lower)

[Table 1]

| Compound | Starting Material | AO | a | b | c | a+b+c | n | y | x |
|---|---|---|---|---|---|---|---|---|---|
| 1 | Butanol | Oxypropylene group | 32 | 6 | 37 | 75 | 4 | 40 | 0.57 |
| 2 | Dodecanol | Oxybutylene group | 8 | 24 | 55 | 87 | 12 | 32 | 0.95 |
| 3 | Hexadecanol | Oxypropylene group | 4 | 4 | 20 | 28 | 16 | 36 | 0.86 |
| 4 | 1-Methylheptadecanol | Oxypropylene group | 2 | 2 | 20 | 24 | 18 | 38 | 0.92 |
| 5 | 2-Decyltetradecanol | Oxypropylene group | 8 | 5 | 24 | 37 | 24 | 56 | 0.78 |
| 6 | 2-Tetradecyloctadecanol | Oxypropylene group | 3 | 5 | 28 | 36 | 32 | 67 | 0.92 |
| 7 | 2-Hexadecyleicosanol | Oxypropylene group | 5 | 10 | 32 | 47 | 36 | 77 | 0.89 |
| 8 | 2-Decyltetradecanol | Oxypropylene group | 10 | 5 | 24 | 39 | 24 | 58 | 0.74 |
| 9 | 2-Decyltetradecanol | Oxypropylene group | 5 | 8 | 24 | 37 | 24 | 53 | 0.86 |
| 10 | Tridecanol | Oxypropylene group | 8 | 5 | 24 | 37 | 13 | 34 | 0.78 |
| 11 | 2-Decyltetradecanol | Oxypropylene group | 1 | 12 | 24 | 37 | 24 | 49 | 0.97 |
| 12 | 2-Decyltetradecanol | Oxypropylene group | 10 | 10 | 10 | 30 | 24 | 58 | 0.67 |
| 13 | Decanol | Oxypropylene group | 8 | 5 | 24 | 37 | 10 | 28 | 0.78 |
| 14 | 2-Decyltetradecanol | Oxypropylene group | 13 | 0 | 24 | 37 | 24 | 61 | - |
| 15 | 2-Decyltetradecanol | Oxypropylene group | 0 | 13 | 24 | 37 | 24 | 48 | 1.00 |
| 16 | Lauryl alcohol | Oxypropylene group | 3 | 2 | 5 | 10 | 12 | 27 | 0.70 |
| 17 | Z-{O-[(BO)$_{7.5}$-(EO)$_{20}$]-H}$_2$ Z is the residue obtained by removing the hydroxyl groups from dimer diol, and BO is an oxybutylene group | | | | | | | | |

(continued)

| Compound | Starting Material | AO | a | b | c | a+b+c | n | y | x |
|---|---|---|---|---|---|---|---|---|---|
| 18 | Polyoxyethylene (60) hydrogenated castor oil ("Uniox HC-60" manufactured by NOF Corporation) | | | | | | | | |
| 19 | Polyoxyethylene sorbitan monooleate (20 E.O.) ("Nonion OT-221" manufactured by NOF Corporation) | | | | | | | | |

Examples 1 to 13 and Comparative Examples 1 to 4 using compounds 1 to 17 mentioned above were used to confirm the solubilized state and evaluate the long-term stability of solubilized liquid and the wetting effect. The results of the confirmation of the solubilized state and the long-term stability of the solubilized liquid are summarized in Table 2-1, and the results of wetting effect are summarized in Table 2-2, respectively. Further, the freezing points of compouds 1 to 17 mentioned above are also shown in Tables 2-1 and 2-2. Note that the freezing points were measured by a method according to JIS K-0065-1992.

(a) Confirmation of Solubilized State

To evaluate the solubilizing power of the compounds of Examples 1 to 11 and Comparative Examples 1 to 3, solubilized liquids comprising the following substances were prepared. The solubilized state was confirmed according to the following method. That is, the transmittance (% T) at 25°C and at 600 nm was measured using an ultraviolet visible light spectrophotometer (V-530, JASCO) and evaluated as follows.
Excellent: Transmittance of 95% or more
Good: Transmittance of 90% or more and less than 95%
Fair: Transmittance of 50% or more and less than 90%
Poor: Transmittance of less than 50%

Solubilized Liquid

| | |
|---|---|
| Compounds 1 to 11 and 14 to 16: | 4% |
| Liquid paraffin *1): | 1% |
| Water | 95% |
| *1: Moresco White P-70, MORESCO Corporation | |

(b) Long-Term Stability of Solubilized Liquid

50 mL of the solubilized liquid described above were poured into transparent glass containers, the containers were sealed and stored at 0°C, 25°C, and 40°C for one month. After one month, the appearances of the solubilized liquids stored at the temperatures were observed and judged according to the following criteria.
Good: The appearance of the solubilized liquids after storage at all of the temperatures was colorless and transparent, and there was no change.
Poor: The appearance of the solubilized liquids after storage at any of the temperature was semi-transparent or cloudy, and there were changes such as separation.

(c) Wetting Effect

To evaluate the wetting power of a wetting agent for the compounds of Examples 1 to 13 and Comparative Examples 1 and 4, a 5% aqueous solution of each of the compounds was used to measure the contact angle (measurement device: DM-501, Drop Mater series, manufactured by Kyowa Interface Science Co., Ltd.) with respect to a glass plate (MICRO SLIDE GLASS, Matsunami Glass Ind., Ltd.), a cold-rolled steel sheet (SPCC-SD, JIS G3141), and an artificial leather (SAPURARE PBZ13001, Ideatex Japan Co., Ltd.). The test solution was filled into a glass syringe attached with a Teflon-coated needle 18G, the droplet amount was adjusted to 2.0 μL, and the contact angle was measured 10 seconds after the droplet deposition.
Note that the measurement was performed five times for each sample, and the average value of these measurements was used as the value of the contact angle. The contact angle of water was used as a blank, the contact angles of each of the compounds (5% aqueous solution) with respect to various types of substrates were compared, and the reduction rate of the contact angle was calculated by the following equation. Note that evaluation was performed according to the following criteria.

$$\text{Contact angle reduction rate (\%)} = \{1 - (\text{contact angle of test compound})/(\text{contact angle of blank})\} * 100$$

Excellent: The reduction rate of the contact angle is 25% or more with respect to all of the substrates.
Good: The contact angle reduction rate is 15% or more and less than 25% with respect to all of the substrates.
Fair: The contact angle reduction rate is less than 15% with respect to any of the substrates.
Poor: The contact angle reduction rate is less than 15% with respect to all of the substrates.

[Table 2-1]

|  | Compound | Freezing Point | Solubilized State | Long-Term Stability of Solubilized Liquid |
|---|---|---|---|---|
| Example 1 | 1 | 0° C or lower | Good | Good |
| Example 2 | 2 | 0° C or lower | Good | Good |
| Example 3 | 3 | 4° C | Good | Good |
| Example 4 | 4 | 3° C | Good | Good |
| Example 5 | 5 | 5° C | Excellent | Good |
| Example 6 | 6 | 7° C | Excellent | Good |
| Example 7 | 7 | 6° C | Excellent | Good |
| Example 8 | 8 | 9° C | Excellent | Good |
| Example 9 | 9 | -20° C or lower | Excellent | Good |
| Example 10 | 10 | 2° C | Good | Good |
| Example 11 | 11 | -20° C or lower | Good | Good |
| Comparative Example 1 | 14 | 38° C | Good | Poor |
| Comparative Example 2 | 15 | 0° C or lower | Fair | Poor |
| Comparative Example 3 | 16 | 0° C or lower | Poor | Poor |

[Table 2-2]

|  | Compound | Freezing Point | Wettability |
|---|---|---|---|
| Example 1 | 1 | 0° C or lower | Excellent |
| Example 2 | 2 | 0° C or lower | Good |
| Example 3 | 3 | 4° C | Excellent |
| Example 4 | 4 | 3° C | Good |
| Example 5 | 5 | 5° C | Excellent |
| Example 6 | 6 | 7° C | Excellent |
| Example 7 | 7 | 6° C | Good |
| Example 8 | 8 | 9° C | Excellent |
| Example 9 | 9 | -20° C or lower | Excellent |
| Example 10 | 10 | 2° C | Excellent |
| Example 11 | 11 | -20° C or lower | Good |
| Example 12 | 12 | 0° C or lower | Good |

(continued)

|  | Compound | Freezing Point | Wettability |
|---|---|---|---|
| Example 13 | 13 | 0° C or lower | Excellent |
| Comparative Example 1 | 14 | 38° C | Fair |
| Comparative Example 4 | 17 | 40° C | Poor |

Examples 14 to 23 and Comparative Examples 5 to 12

Compounds 5, 8 to 11, 14, 18, and 19 listed in Table 1, the following fragrances, and water were mixed at room temperature (25°C) in the amounts shown in Tables 3-1 and 3-2 until the mixture was uniform to obtain a liquid solubilizing composition.

Fragrances

(R)-(+)-Limonene (manufactured by Wako Pure Chemical Industries, Ltd.)
trans-Anethole (manufactured by Wako Pure Chemical Industries, Ltd.)
Ethyl hexanoate (manufactured by Wako Pure Chemical Industries, Ltd.)
1-Nonanol (manufactured by Tokyo Chemical Industry Co., Ltd.)
p-Anisaldehyde (manufactured by Tokyo Chemical Industry Co., Ltd.)
Caprylic acid (manufactured by Wako Pure Chemical Industries, Ltd.)

Evaluation

Confirmation of the solubilized state and evaluation of the stickiness during application and the fragrance development were performed for the obtained solubilizing compositions of Examples 14 to 23 and Comparative Examples 5 to 12 as described below. The results are shown in Tables 3-1 and 3-2.

(a) Confirmation of Solubilized State

Confirmation that the solubilizing composition was in a solubilized state was performed in accordance with the following method. That is, the transmittance (%) of a 1 cm cell of visible light having a wavelength of 600 nm was measured at 25°C using an ultraviolet visible light spectrophotometer (V-530, JASCO) and evaluated as follows.
Excellent: Transmittance of 95% or more
Good: Transmittance of 80% or more and less than 95%
Fair: Transmittance of 50% or more and less than 80%
Poor: Transmittance of less than 50%

(b) Stickiness during Application

0.2 mL of the solubilizing composition was applied to the forearms of 10 panelists, and sensory evaluation of the stickiness of the preparation was performed according to the following criteria.

3 points: The panelist felt almost no stickiness.
2 points: The panelist felt some stickiness.
1 point: The panelist felt strong stickiness.

Further, the total score of the evaluation of the 10 panelists was determined according to the following criteria.
Excellent: 25 points or more (the solubilizing composition has almost no stickiness)
Good: 20 points or more and less than 25 points (the solubilizing composition is somewhat sticky)
Fair: 15 points or more and less than 20 points (the solubilizing composition has stickiness)
Poor: less than 15 points (the solubilizing composition has strong stickiness)

(c) Fragrance Development

0.2 mL of the solubilizing composition was applied to the forearms of 10 panelists, and sensory evaluation of the strength of fragrance development was performed according to the following criteria.

3 points: The panelist perceived a strong fragrance development.
2 points: The panelist perceived some fragrance development.
1 point: The panelist perceived almost no fragrance development.

Further, the total score of the evaluation of the 10 panelists was determined according to the following criteria.
Excellent: 25 points or more (the solubilizing composition has extremely excellent fragrance development)
Good: 20 points or more and less than 25 points (the solubilizing composition has excellent fragrance development)
Fair: 15 points or more and less than 20 points (the solubilizing composition has somewhat excellent fragrance development)
Poor: less than 15 points (the solubilizing composition has poor fragrance development)

[Table 3-1]

| | Examples | | | | | | | | | |
|---|---|---|---|---|---|---|---|---|---|---|
| | 14 | 15 | 16 | 17 | 18 | 19 | 20 | 21 | 22 | 23 |
| Compound 5 (%) | 2 | 2 | 2 | 2 | 2 | 2 | - | - | - | - |
| Compound 8 (%) | - | - | - | - | - | - | 2 | - | - | - |
| Compound 9 (%) | - | - | - | - | - | - | - | 2 | - | - |
| Compound 10 (%) | - | - | - | - | - | - | - | - | 2 | - |
| Compound 11 (%) | - | - | - | - | - | - | - | - | - | 2 |
| (R)-(+)-Limonene (%) | 0.5 | - | - | - | - | - | - | - | - | - |
| trans-Anethole (%) | - | 0.5 | - | - | - | - | - | - | - | - |
| Ethyl hexanoate (%) | - | - | 0.5 | - | - | - | - | - | - | - |
| 1-Nonanol (%) | - | - | - | 0.5 | - | - | 0.5 | 0.5 | 0.5 | 0.5 |
| p-Anisaldehyde (%) | - | - | - | - | 0.5 | - | - | - | - | - |
| Caprylic acid (%) | - | - | - | - | - | 0.5 | - | - | - | - |
| Water | Remaining Part | | | | | | | | | |
| Total (%) | 100 | 100 | 100 | 100 | 100 | 100 | 100 | 100 | 100 | 100 |
| Confirmation of Solubilized State | Excellent | Excellent | Excellent | Excellent | Excellent | Excellent | Excellent | Excellent | Good | Good |
| Stickiness during Application | Excellent | Excellent | Excellent | Excellent | Excellent | Excellent | Good | Excellent | Excellent | Good |
| | 29 | 26 | 28 | 25 | 28 | 27 | 24 | 27 | 26 | 23 |
| Fragrance Development of Preparation | Excellent | Excellent | Excellent | Excellent | Excellent | Excellent | Excellent | Good | Good | Good |
| | 25 | 27 | 29 | 26 | 29 | 26 | 26 | 21 | 24 | 22 |

[Table 3-2]

| | Comparative Examples | | | | | | | |
|---|---|---|---|---|---|---|---|---|
| | 5 | 6 | 7 | 8 | 9 | 10 | 11 | 12 |
| Compound 14 | 2 | 2 | 2 | 2 | 2 | 2 | - | - |
| Compound 18 | - | - | - | - | - | - | 2 | - |
| Compound 19 | - | - | - | - | - | - | - | 2 |
| (R)-(+)-Limonene (%) | 0.5 | - | - | - | - | - | - | - |
| trans-Anethole (%) | - | 0.5 | - | - | - | - | - | - |
| Ethyl hexanoate (%) | - | - | 0.5 | - | - | - | - | - |
| 1-Nonanol (%) | - | - | - | 0.5 | - | - | 0.5 | 0.5 |
| p-Anisaldehyde (%) | - | - | - | - | 0.5 | - | - | - |
| Caprylic acid (%) | - | - | - | - | - | 0.5 | - | - |
| Water | Remaining Part | | | | | | | |
| Total (%) | 100 | 100 | 100 | 100 | 100 | 100 | 100 | 100 |
| Confirmation of Solubilized State | Poor | Poor | Good | Fair | Excellent | Poor | Excellent | Poor |
| Stickiness during Application | Excellent | Excellent | Excellent | Excellent | Excellent | Excellent | Poor | Fair |
| | 27 | 26 | 28 | 25 | 25 | 26 | 14 | 19 |
| Fragrance Development of Preparation | Poor | Fair | Poor | Fair | Poor | Fair | Poor | Good |
| | 14 | 21 | 13 | 17 | 12 | 19 | 12 | 21 |

As is apparent from Table 3-1, each of the solubilizing compositions of Examples 14 to 23 had excellent performance.

In some cases, the fragrance could not be solubilized or the fragrance development was weak in Comparative Examples 5 to 10 using a component which did not satisfy the requirements of component (B) according to the present invention, that is, compound 14 for which the random rate x was outside the range specified in the present invention.

In Comparative Example 11 in which compound 18 was used, the fragrance could be solubilized, however, strong stickiness occurred during application and the fragrance development was also weak.

In Comparative Example 12 in which compound 19 was used, the fragrance could not be solubilized and some stickiness occurred during application.

Example 24

If the above-mentioned compound 5 and the following components are mixed in the amounts shown in Table 4 at room temperature (25°C) until the mixture is uniform, it is possible to obtain a liquid solubilizing composition (skin lotion) which has excellent transparency, little stickiness during application, and excellent fragrance development.

[Table 4]

| | Composition |
| --- | --- |
| Compound 5 | 0.50% |
| Fragrance | 0.10% |
| Glycerol | 3.00% |
| 1,3-Butylene glycol | 5.00% |
| Polyquaternium-51 (5% aqueous solution) | 1.00% |
| Methyl Gluceth-10 | 1.00% |
| Ethylhexylglycerol | 0.01% |
| Citric acid | 0.05% |
| Sodium citrate | 0.02% |
| Tocopherol (d-$\delta$-tocopherol) | 0.05% |
| Disodium EDTA | 0.01% |
| Water | Remaining Part |
| Total | 100% |

Example 25

The above-mentioned compound 5 and the following components are mixed at 80°C in the amounts shown in Table 5 to obtain a liquid solubilizing composition (body soap) having excellent transparency, little stickiness during use, and excellent fragrance development.

[Table 5]

| | Composition |
| --- | --- |
| Compound 5 | 2.00% |
| Fragrance | 0.50% |
| Hydroxyethyl cellulose | 0.50% |
| Glycerol | 5.00% |
| PG | 8.00% |
| Lauric acid | 9.00% |
| Myristic acid | 5.00% |

(continued)

| | Composition |
|---|---|
| Palmitic acid | 3.00% |
| Potassium hydroxide (48% aqueous solution) | 9.50% |
| Potassium taurine laurate | 6.00% |
| Disodium EDTA | 0.02% |
| Water | Remaining Part |
| Total | 100% |

Example 26

The above-mentioned compound 5 and the following components are mixed at 80°C in the amounts shown in Table 6 to obtain a liquid solubilizing composition (shampoo) having excellent transparency, little stickiness during use, and excellent fragrance development.

[Table 6]

| | Composition |
|---|---|
| Compound 5 | 2.00% |
| Fragrance | 1.00% |
| Polyquaternium-10 | 0.50% |
| Cocamidopropyl betaine | 6.00% |
| Sodium laureth sulfate | 2.50% |
| Sodium methyl cocoyl taurate | 3.00% |
| Sodium lauroyl methylalanine | 3.00% |
| PEG-120 Methyl glucose triisostearate | 0.75% |
| PG laurate | 0.50% |
| Citric acid | 0.05% |
| Sodium citrate | 0.02% |
| Tocopherol (d-$\delta$-tocopherol) | 0.05% |
| Disodium EDTA | 0.01% |
| Water | Remaining Part |
| Total | 100% |

Example 27

The above-mentioned compound 1 and the following components are mixed at room temperature in the amounts shown in Table 7 until the mixture is uniform to obtain a liquid solubilizing composition (skin cleanser) having excellent transparency, little stickiness during use, and excellent fragrance development.

[Table 7]

| | Composition |
|---|---|
| Compound 1 | 2.00% |
| Fragrance | 0.50% |
| BG | 3.00% |

(continued)

| | Composition |
|---|---|
| PEG-8 glyceryl caprylate/caprate | 3.00% |
| Citric acid | 0.05% |
| Sodium citrate | 0.02% |
| Tocopherol (d-$\delta$-tocopherol) | 0.05% |
| Disodium EDTA | 0.01% |
| Water | Remaining Part |
| Total | 100% |

Example 28

The above-mentioned compound 8 and the following components are mixed at 80°C in the amounts shown in Table 8 until the mixture is uniform to obtain a liquid solubilizing composition (hair mist) having excellent transparency, little stickiness during use, and excellent fragrance development.

[Table 8]

| | Composition |
|---|---|
| Compound 8 | 2.00% |
| Fragrance | 0.50% |
| Ethanol | 5.00% |
| BG | 1.00% |
| Glycerol | 1.00% |
| Polyquaternium-61 | 0.05% |
| Dipotassium glycyrrhizinate | 0.05% |
| Citric acid | 0.05% |
| Sodium citrate | 0.02% |
| Tocopherol (d-$\delta$-tocopherol) | 0.05% |
| Disodium EDTA | 0.01% |
| Water | Remaining Part |
| Total | 100% |

Example 29

The above-mentioned compound 12 and the following components are mixed at ordinary temperature in the amounts shown in Table 9 until the mixture is uniform to obtain a liquid solubilizing composition (aromatic agent) having excellent transparency and excellent fragrance development.

[Table 9]

| | Composition |
|---|---|
| Compound 12 | 4.00% |
| Fragrance | 1.00% |
| Ethanol | 20.00% |
| Disodium EDTA | 0.01% |

(continued)

|  | Composition |
| --- | --- |
| Water | Remaining Part |
| Total | 100% |

Example 30

The above-mentioned compound 12 and the following components are mixed at ordinary temperature in the amounts shown in Table 10 until the mixture is uniform to obtain a liquid solubilizing composition (deodorant agent) having excellent transparency, little stickiness, and excellent fragrance development.

[Table 10]

|  | Composition |
| --- | --- |
| Compound 12 | 2.00% |
| Fragrance | 0.10% |
| Ethanol | 10.00% |
| PPG-52 Butyl ether | 5.00% |
| Benzalkonium chloride | 0.05% |
| Menthol | 0.50% |
| Disodium EDTA | 0.01% |
| Water | Remaining Part |
| Total | 100% |

Examples 31 to 38 and Comparative Examples 13 to 18

Compounds 5, 8 to 11, 14, 18, and 19 listed in Table 1, unsaturated compounds, and water were mixed at room temperature (25°C) in the amounts shown in Tables 11-1 and 11-2 until the mixture was uniform to obtain a liquid solubilizing composition.

Unsaturated Compounds

Retinol ("Retinol 15D" manufactured by BASF)
Tocopherol ("E-Mix D" manufactured by Tama Biochemical Co., Ltd.)
Oleic acid ("EXTRASOME OS-85" manufactured by NOF Corporation)
Linoleic acid ("Linoleic acid 90" manufactured by NOF Corporation)

Evaluation

Confirmation of the solubilized state and evaluation of the stickiness during application and the long-term stability were performed for the obtained solubilizing compositions of Examples 31 to 38 and Comparative Examples 13 to 18 as described below. The results are shown in Tables 11-1 and 11-2.

(a) Confirmation of Solubilized State

Confirmation that the solubilizing composition was in a solubilized state was performed in accordance with the following method. That is, the transmittance (%) of a 1 cm cell of visible light having a wavelength of 600 nm was measured at 25°C using an ultraviolet visible light spectrophotometer (V-530, JASCO) and evaluated as follows.
Excellent: Transmittance of 95% or more
Good: Transmittance of 80% or more and less than 95%
Fair: Transmittance of 50% or more and less than 80%
Poor: Transmittance of less than 50%

(b) Stickiness during Application

0.2 mL of the solubilizing composition was applied to the forearms of 10 panelists, and sensory evaluation of the stickiness of the preparation was performed according to the following criteria.

3 points: The panelist felt almost no stickiness.
2 points: The panelist felt some stickiness.
1 point: The panelist felt strong stickiness.

Further, the total score of the evaluation of the 10 panelists was determined according to the following criteria.
Excellent: 25 points or more (the solubilizing composition has almost no stickiness)
Good: 20 points or more and less than 25 points (the solubilizing composition is somewhat sticky)
Fair: 15 points or more and less than 20 points (the solubilizing composition has stickiness)
Poor: less than 15 points (the solubilizing composition has strong stickiness)

(c) Long-Term Stability of Solubilizing Composition

50 mL of the solubilizing composition were poured into transparent glass containers, the containers were sealed and stored at 0°C, 25°C, and 40°C for one month. After one month, the appearances of the solubilizing compositions stored at the temperatures were observed and judged according to the following criteria.
Good: The appearance of the solubilizing composition after storage at all of the temperatures was colorless and transparent, and there was no change.
Poor: The appearance of the solubilizing composition after storage at any of the temperatures was semi-transparent or cloudy, and there were changes such as precipitation, solidification, and separation.

[Table 11-1]

| | Examples | | | | | | | |
|---|---|---|---|---|---|---|---|---|
| | 31 | 32 | 33 | 34 | 35 | 36 | 37 | 38 |
| Compound 5 (%) | 2 | 2 | 2 | 2 | - | - | - | - |
| Compound 8 (%) | - | - | - | - | 2 | - | - | - |
| Compound 9 (%) | - | - | - | - | - | 2 | - | - |
| Compound 10 (%) | - | - | - | - | - | - | 2 | - |
| Compound 11 (%) | - | - | - | - | - | - | - | 2 |
| Retinol (%) | 0.5 | - | - | - | - | - | - | - |
| Tocopherol (%) | - | 0.5 | - | - | 0.5 | 0.5 | 0.5 | 0.5 |
| Oleic acid (%) | - | - | 0.5 | - | - | - | - | - |
| Linoleic acid (%) | - | - | - | 0.5 | - | - | - | - |
| Water | Remaining Part | | | | | | | |
| Total (%) | 100 | 100 | 100 | 100 | 100 | 100 | 100 | 100 |
| Confirmation of Solubilized State | Excellent | Excellent | Excellent | Excellent | Excellent | Excellent | Excellent | Good |

(continued)

| | Examples | | | | | | | |
|---|---|---|---|---|---|---|---|---|
| | 31 | 32 | 33 | 34 | 35 | 36 | 37 | 38 |
| Stickiness during Application | Excellent | Excellent | Excellent | Excellent | Excellent | Good | Excellent | Good |
| | 28 | 29 | 25 | 26 | 25 | 24 | 26 | 21 |
| Long-Term Stability of Solubilizing Composition | Good | Good | Good | Good | Good | Good | Good | Good |

[Table 11-2]

| | Comparative Examples | | | | | |
|---|---|---|---|---|---|---|
| | 13 | 14 | 15 | 16 | 17 | 18 |
| Compound 14 (%) | 2 | 2 | 2 | 2 | - | - |
| Compound 18 (%) | - | - | - | - | 2 | - |
| Compound 19 (%) | - | - | - | - | - | 2 |
| Retinol (%) | 0.5 | - | - | - | - | - |
| Tocopherol (%) | - | 0.5 | - | - | 0.5 | 0.5 |
| Oleic acid (%) | - | - | 0.5 | - | - | - |
| Linoleic acid (%) | - | - | - | 0.5 | - | - |
| Water | Remaining Part | | | | | |
| Total (%) | 100 | 100 | 100 | 100 | 100 | 100 |
| Confirmation of Solubilized State | Poor | Poor | Good | Fair | Good | Poor |
| Stickiness during Application | Excellent | Excellent | Excellent | Excellent | Poor | Fair |
| | 26 | 29 | 26 | 27 | 14 | 17 |
| Long-Term Stability of Solubilizing Composition | Poor | Good | Poor | Poor | Poor | Good |

As is apparent from Table 11-1, each of the solubilizing compositions of Examples 31 to 38 had excellent performance.

In some cases, the unsaturated compound could not be solubilized or the long-term stability was low in Comparative Examples 13 to 16 using a component which did not satisfy the requirements of component (B) according to the present invention, that is, compound 14 for which the random rate x was outside the range specified in the present invention.

In Comparative Example 17 in which compound 18 was used, the unsaturated compound could be solubilized, however, there was a case where strong stickiness occurred during application and the long-term stability was low.

In Comparative Example 18 in which compound 19 was used, the unsaturated compound could not be solubilized and some stickiness occurred during application.

Example 39

If the above-mentioned compound 5 and the following components are mixed at room temperature (25°C) in the amounts shown in Table 12 until the mixture is uniform, it is possible to obtain a solubilizing composition (beauty serum) which has excellent transparency, little stickiness during application, and excellent long-term stability.

[Table 12]

|  | Composition |
| --- | --- |
| Compound 5 | 0.50% |
| Retinol | 0.10% |
| Glycerol | 3.00% |
| 1,3-Butylene glycol | 5.00% |
| Polyquaternium-51 (5% aqueous solution) | 1.00% |
| Methyl Gluceth-10 | 1.00% |
| Ethylhexylglycerol | 0.01% |
| Citric acid | 0.05% |
| Sodium citrate | 0.02% |
| Tocopherol (d-$\delta$-tocopherol) | 0.05% |
| Disodium EDTA | 0.01% |
| Water | Remaining Part |
| Total | 100% |

Examples 40 to 48 and Comparative Examples 19 to 25

The following components (A-1) and (A-2), compounds 5, 8 to 11, and 14 listed in Table 1, the following components (C-1) to (C'-2), the following component (D), other components, and water were mixed at room temperature (25°C) in the amounts shown in Tables 14-1 and 14-2 until the mixture was uniform to obtain a liquid solubilizing composition.

Components (A): Saturated Compounds

Component (A-1): Squalane (Super Squalane, JX Nippon Oil & Energy Trading Corporation) (octanol/water partition coefficient: 12.5, molar mass: 422 g/mol)
Component (A-2): Liquid paraffin (Moresco White P-70, MORESCO Corporation) (octanol/water partition coefficient: 10.1, molar mass: 324 g/mol)

Synthesis of Components (C-1) to (C'-2)

An alkylene oxide derivative (components (C-1) to (C'-2)) was synthesized by a method similar to Synthesis Example 1, except that the type of the starting materials and the addition amounts of ethylene oxide, propylene oxide, and 1,2-butylene oxide were changed.
Table 13 shows the starting materials (Z-(OH)s) used for the synthesis of components (C-1) to (C'-2), the amount of PO with respect to a total of 100 parts by mass of s, p, q, r, PO, and EO in formula (II) of the obtained components (C-1) to (C'-2), and the cloud point of the 5% aqueous solutions.

[Table 13]

| Component | Starting Material | s | p | q | r | Mass fraction of PO | Cloud point of 5% aqueous solution (°C) |
| --- | --- | --- | --- | --- | --- | --- | --- |
| (C-1) | Ethylene glycol | 2.0 | 2.5 | 4.0 | 1.0 | 45.2 | 42 |
| (C-2) | Glycerol | 3.0 | 1.7 | 2.7 | 1.0 | 45.2 | 36 |
| (C-3) | Methylglucoside | 4.0 | 1.8 | 5.0 | 1.0 | 31.6 | 28 |
| (C'-1) | Ethylene glycol | 2.0 | 0 | 5.0 | 0 | 0 | - |
| (C'-2) | Propylene glycol | 2.0 | 5.0 | 0 | 0 | 100.0 | - |

Component (D)

Component (D): ethylhexylglycerol (3-(2-ethylhexyloxy)-1,2-propanediol manufactured by SACHEM, Inc.)

Evaluation

Confirmation of the solubilized state and evaluation of the stickiness during application and the long-term stability were performed for the obtained solubilizing compositions of Examples 40 to 48 and Comparative Examples 19 to 25 as described below. The results are shown in Tables 14-1 and 14-2.

(a) Confirmation of Solubilized State

Confirmation that the solubilizing composition was in a solubilized state was performed in accordance with the following method. That is, the transmittance (%) of a 1 cm cell of visible light having a wavelength of 600 nm was measured at 25°C using an ultraviolet visible light spectrophotometer (V-530, JASCO) and evaluated as follows.
Excellent: Transmittance of 90% or more
Good: Transmittance of 80% or more and less than 90%
Fair: Transmittance of 50% or more and less than 80%
Poor: Transmittance of less than 50%

(b) Stickiness during Application

0.2 mL of the solubilizing composition was applied to the forearms of 10 panelists, and sensory evaluation of the stickiness of the preparation was performed according to the following criteria.

3 points: The panelist felt almost no stickiness.
2 points: The panelist felt some stickiness.
1 point: The panelist felt strong stickiness.

Further, the total score of the evaluation by the 10 panelists was determined according to the following criteria.
Excellent: 25 points or more (the solubilizing composition has almost no stickiness)
Good: 20 points or more and less than 25 points (the solubilizing composition is somewhat sticky)
Fair: 15 points or more and less than 20 points (the solubilizing composition has stickiness)
Poor: less than 15 points (the solubilizing composition has strong stickiness)

(c) Long-Term Stability of Solubilizing Composition

50 mL of the solubilizing composition were poured into transparent glass containers, the containers were sealed and stored at 0°C, 25°C, and 40°C for one month. After one month, the appearances of the solubilizing compositions stored at the temperatures were observed and judged according to the following criteria.
Good: The appearance of the solubilizing composition after storage at all of the temperatures was colorless and transparent, and there was no change.
Poor: The appearance of the solubilizing composition after storage at any of the temperatures was semi-transparent or cloudy, and there were changes such as precipitation, solidification, and separation.

EP 3 632 959 A1

[Table 14-1]

| | Examples | | | | | | | | |
|---|---|---|---|---|---|---|---|---|---|
| | 40 | 41 | 42 | 43 | 44 | 45 | 46 | 47 | 48 |
| Component (A-1) (%) | 0.5 | 0.5 | 0.5 | 0.5 | 0.5 | 0.5 | 0.5 | - | 2.1 |
| Component (A-2) (%) | - | - | - | - | - | - | - | 0.5 | - |
| Compound 5 (%) | 3 | - | - | - | - | 3 | 3 | 3 | 2.7 |
| Compound 8 (%) | - | 3 | - | - | - | - | - | - | - |
| Compound 9 (%) | - | - | 3 | - | - | - | - | - | - |
| Compound 10 (%) | - | - | - | 3 | - | - | - | - | - |
| Compound 11 (%) | - | - | - | - | 3 | - | - | - | - |
| Component (C-1) (%) | - | - | - | - | - | 5 | - | - | - |
| Component (C-2) (%) | 5 | 5 | 5 | 5 | 5 | - | - | 5 | 0.2 |
| Component (C-3) (%) | - | - | - | - | - | - | 5 | - | - |
| Component (D) (%) | 0.3 | 0.3 | 0.3 | 0.3 | 0.3 | 0.3 | 0.3 | 0.3 | 0.3 |
| Glycerol | - | - | - | - | - | - | - | - | 20 |
| Isostearyl alcohol | - | - | - | - | - | - | - | - | 0.8 |
| Water | Remaining Part | | | | | | | | |
| Total (%) | 100 | 100 | 100 | 100 | 100 | 100 | 100 | 100 | 100 |
| Mass of component (B)/mass of component (A) | 6 | 6 | 6 | 6 | 6 | 6 | 6 | 6 | 1.3 |
| Confirmation of Solubilized State | Excellent | Excellent | Good | Excellent | Good | Good | Good | Excellent | Excellent |
| Stickiness during Application | Excellent | Good | Excellent | Good | Excellent | Excellent | Excellent | Excellent | Excellent |
| | 28 | 23 | 28 | 24 | 28 | 27 | 26 | 27 | 29 |
| Long-Term Stability of Preparation | Good | Good | Good | Good | Good | Good | Good | Good | Good |

26

[Table 14-2]

| | Comparative Examples | | | | | | |
|---|---|---|---|---|---|---|---|
| | 19 | 20 | 21 | 22 | 23 | 24 | 25 |
| Component (A-1) (%) | 0.5 | 0.5 | 0.5 | 2 | 0.5 | 0.5 | 0.5 |
| Compound 5 (%) | 3 | 3 | 10 | 0.5 | - | 3 | 3 |
| Compound 14 (%) | - | - | - | - | 3 | - | - |
| Component (C-2) (%) | - | 5 | 5 | 5 | 5 | - | - |
| Component (C'-1) (%) | - | - | - | - | - | 5 | - |
| Component (C'-2) (%) | - | - | - | - | - | - | 5 |
| Component (D) (%) | - | - | 0.3 | 0.3 | 0.3 | 0.3 | 0.3 |
| Water | Remaining Part | | | | | | |
| Total (%) | 100 | 100 | 100 | 100 | 100 | 100 | 100 |
| Mass of component (B)/mass of component (A) | 6 | 6 | 20 | 0.25 | 6 | 6 | 6 |
| Confirmation of Solubilized State | Poor | Poor | Excellent | Poor | Poor | Fair | Poor |
| Stickiness during Application | Excellent | Excellent | Poor | Good | Excellent | Good | Good |
| | 25 | 27 | 14 | 23 | 25 | 24 | 22 |
| Long-Term Stability of Preparation | Poor | Poor | Good | Poor | Poor | Poor | Poor |

As is apparent from Table 14-1, each of the solubilizing compositions of Examples 40 to 48 had excellent performance.

Conversely, as is apparent from Table 14-2, in the solubilizing compositions of Comparative Examples 19 and 20 in which components (C) and/or component (D) according to the present invention were not used, component (A) could not be solubilized, and the long-term stability was low.

Further, in the solubilizing compositions of Comparative Examples 21 and 22 in which the ratio of the mass of component (B) to the mass of component (A) (the mass of component (B)/the mass of component (A)) was outside the range from 0.5 to 10, either stickiness or long-term stability was inferior compared to the solubilizing compositions of Examples 40 to 48.

In Comparative Example 23 using a component which did not satisfy the requirements of component (B) according to the present invention, that is, compound 14 for which the random rate x was outside the range specified in the present invention, component (A) could not be solubilized, and the long-term stability was low.

In the solubilizing compositions of Comparative Examples 24 and 25 in which a component was used that did not satisfy the requirements of component (C) according to the present invention, there were cases where component (A) could not be solubilized, and the long-term stability was low.

## Claims

1. An alkylene oxide derivative represented by following formula (I):

$$R\text{-O-}(AO)_a\text{-}[(PO)_b/(EO)_c]\text{-H} \dots \qquad (I)$$

(R represents a linear or branched alkyl group having from 4 to 36 carbons; AO is an oxyalkylene group having three or four carbons, PO is an oxypropylene group, and EO is an oxyethylene group; a, b, and c are the average addition molar numbers of the oxyalkylene group, the oxypropylene group, and the oxyethylene group per molecule, respectively, and $1 \leq a \leq 40$, $1 \leq b \leq 40$, $1 \leq c \leq 80$, and $(a + b + c) \geq 20$; $[(PO)_b/(EO)_c]$ represents a polyoxyalkylene group in which b moles of PO and c moles of EO are bonded randomly, and the random rate x is $0.1 \leq x < 1$).

2. A wetting agent comprising the alkylene oxide derivative according to claim 1.

3. A solubilizing agent for solubilizing an oily component in water, the solubilizing agent comprising the alkylene oxide derivative according to claim 1.

4. The solubilizing agent according to claim 3, wherein the oily component is a fragrance.

5. The solubilizing agent according to claim 3, wherein the oily component is an unsaturated compound.

6. The solubilizing agent according to claim 3, wherein the oily component is a saturated compound having an octanol/water partition coefficient of 7 to 20 and a molar mass of 250 g/mol or more.

7. The solubilizing agent according to claim 4 or 5, wherein, in the alkylene oxide derivative according to claim 1, R is a linear or branched alkyl group having from 12 to 32 carbons, and $1 \leq a \leq 30$, $1 \leq b \leq 30$, and $1 \leq c \leq 60$.

8. A solubilizing composition comprising 0.01 to 5 mass% of component (A), 0.1 to 10 mass% of component (B), 0.1 to 20 mass% of component (C), and 0.01 to 5 mass% of component (D), with the ratio of the mass of component (B) to the mass of component (A) (the mass of component (B)/the mass of component (A)) being 0.5 to 10, wherein the component (A) is a saturated compound having an octanol/water partition coefficient of 7 to 20 and a molar mass of 250 g/mol or more,

the component (B) is the alkylene oxide derivative according to claim 1,

the component (C) is an alkylene oxide derivative represented by the following formula (II) where a 5 mass% aqueous solution of the component (C) has a cloud point of 15°C or higher

$$Z\text{-}\{O\text{-}[(PO)_p/(EO)_q]\text{-}(BO)_r\text{-}H\}_s \ ... \qquad (II)$$

(wherein Z is a residue obtained by removing all hydroxyl groups from a polyhydric alcohol having from two to seven carbons and containing s hydroxyl groups, and $2 \leq s \leq 4$; PO is an oxypropylene group, EO is an oxyethylene group, BO is an oxyalkylene group having four carbons, p, q, and r are the average addition molar numbers of the oxypropylene group, the oxyethylene group, and the oxyalkylene group per molecule, respectively, $1 \leq p \leq 30$, $1 \leq q \leq 30$, $1 \leq r \leq 2$, and PO and EO are bonded randomly), and

the component (D) is an alkyl glyceryl ether comprising glycerol and a monohydric alcohol including a branched alkyl having from seven to nine carbons.

9. The solubilizing composition according to claim 8, wherein, in the alkylene oxide derivative according to claim 1, R is a linear or branched alkyl group having from 12 to 32 carbons, and $1 \leq a \leq 30$, $1 \leq b \leq 30$, and $1 \leq c \leq 60$.

## INTERNATIONAL SEARCH REPORT

International application No.

PCT/JP2018/019389

**A. CLASSIFICATION OF SUBJECT MATTER**
Int.Cl. See extra sheet

According to International Patent Classification (IPC) or to both national classification and IPC

**B. FIELDS SEARCHED**

Minimum documentation searched (classification system followed by classification symbols)
Int.Cl.    C08G65/28, A61K8/45, A61K8/86, A61K8/90, A61Q5/00, A61Q13/00,
           A61Q15/00, A61Q19/00, A61Q19/10, B01F1/00, B01F17/42, C08K5/01,
           C08K5/10, C08L71/02, C11B9/00

Documentation searched other than minimum documentation to the extent that such documents are included in the fields searched
Published examined utility model applications of Japan       1922–1996
Published unexamined utility model applications of Japan     1971–2018
Registered utility model specifications of Japan             1996–2018
Published registered utility model applications of Japan     1994–2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2014-240378 A (MATSUMOTO YUSHI-SEIYAKU CO., | 1-7 |
| Y | LTD.) 25 December 2014, claims, paragraph [0047], | 2-7 |
| A | examples (Family: none) | 8, 9 |
| X | JP 2014-196440 A (DKS CO., LTD.) 16 October 2014, | 1 |
| A | claims, examples (Family: none) | 2-9 |
| X | JP 2015-096247 A (MATSUMOTO YUSHI-SEIYAKU CO., | 1 |
| A | LTD.) 21 May 2015, claims, paragraph [0058], examples, in particular, alkylene oxide adduct 4 (Family: none) | 2-9 |
| X | JP 05-186985 A (LION CORP.) 27 July 1993, claims, | 1 |
| A | examples & JP 08-284087 A & US 5417808 A; claims, examples & CA 2076308 A1 | 2-9 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 August 2018 (09.08.2018) | 21 August 2018 (21.08.2018) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2018/019389

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | JP 2000-265385 A (LION CORP.) 26 September 2000, claims, examples (Family: none) | 1<br>2-9 |
| Y<br>A | JP 2003-226892 A (DKS CO., LTD.) 15 August 2003, claims, claim 5, paragraphs [0045]-[0048], examples (Family: none) | 2-7<br>1, 8, 9 |
| A | JP 2008-247859 A (NOF CORPORATION) 16 October 2008, claims, examples (Family: none) | 1-9 |
| A | JP 2005-281450 A (DKS CO., LTD.) 13 October 2005, claims, examples (Family: none) | 1-9 |
| A | JP 54-021979 A (NIPPON OIL & FATS CO., LTD.) 19 February 1979, claims, examples (Family: none) | 1-9 |
| P, A | JP 2018-030793 A (NOF CORPORATION) 01 March 2018, claims, examples (Family: none) | 1-9 |
| P, A | JP 2017-222605 A (NOF CORPORATION) 21 December 2017, claims, examples (Family: none) | 1-9 |

Form PCT/ISA/210 (continuation of second sheet) (January 2015)

**INTERNATIONAL SEARCH REPORT**

International application No.

PCT/JP2018/019389

CLASSIFICATION OF SUBJECT MATTER
C08G65/28(2006.01)i,        A61K8/45(2006.01)i,        A61K8/86(2006.01)i,
A61K8/90(2006.01)i,        A61Q5/00(2006.01)i,        A61Q13/00(2006.01)i,
A61Q15/00(2006.01)i,        A61Q19/00(2006.01)i,        A61Q19/10(2006.01)i,
B01F1/00(2006.01)i,        B01F17/42(2006.01)i,        C08K5/01(2006.01)i,
C08K5/10(2006.01)i, C08L71/02(2006.01)i, C11B9/00(2006.01)i

Form PCT/ISA/210 (extra sheet) (January 2015)

**EP 3 632 959 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000325771 A
- JP 2008239496 A
- JP 2014240378 A
- JP 2001213718 A
- JP 2008266217 A